(19) Europäisches Patentamt
European Patent Office
Office européen des brevets

(11) Veröffentlichungsnummer: **0 011 849**
**B1**

(12) **EUROPÄISCHE PATENTSCHRIFT**

(45) Veröffentlichungstag der Patentschrift: **19.05.82**

(21) Anmeldenummer: **79104702.0**

(22) Anmeldetag: **27.11.79**

(51) Int. Cl.³: **C 07 C 103/84,**
A 61 K 49/04

# (54) Neue 2,4,6-Trijod-Isophthalamsäurederivate, deren Herstellung und Röntgenkontrastmittel auf Basis dieser Verbindungen.

(30) Priorität: **30.11.78 DE 2852094**

(43) Veröffentlichungstag der Anmeldung:
**11.06.80 Patentblatt 80/12**

(45) Bekanntmachung des Hinweises auf die Patenterteilung:
**19.05.82 Patentblatt 82/20**

(84) Benannte Vertragsstaaten:
**AT BE CH DE FR GB IT LU NL SE**

(56) Entgegenhaltungen:
**DE - A - 2 207 950**
**DE - A - 2 505 320**
**DE - A - 2 523 567**

(73) Patentinhaber: **SCHERING AKTIENGESELLSCHAFT**
**Berlin und Bergkamen**
**Müllerstrasse 170/178 Postfach 65 03 11**
**D-1000 Berlin 65 (DE)**

(72) Erfinder: **Klieger, Erich, Dr.**
**Augsburger Strasse 25**
**D-1000 Berlin 30 (DE)**
Erfinder: **Mützel, Wolfgang, Dr.**
**Weddigenweg 74**
**D-1000 Berlin 45 (DE)**

**EP 0 011 849 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Courier Press, Leamington Spa, England.

# Neue 2,4,6-Trijod-Isophthalamsäurederivate, deren Herstellung und Röntgenkontrastmittel auf Basis dieser Verbindungen

Die Erfindung betrifft die in den Ansprüchen gekennzeichneten Gegenstände.

Der niedere Alkylrest $R_1$, der gegebenenfalls mit einer Methoxy- oder Ethoxygruppe substituiert sein kann, enthält im Alkylrest 1—4 Kohlenstoffatome, vorzugsweise 1—2 Kohlenstoffatome.

Bevorzugte niedere Alkylreste $R_1$ mit 1—2 C-Atomen können durch eine Methoxy- oder Ethoxygruppe substituiert sein wie beispielsweise Methyl, Äthyl, Methoxymethyl.

Unter den niederen Alkylresten $R_2$, $R_3$ und $R_4$ mit 1—4 C-Atomen sind Reste mit 1—2 Kohlenstoffatomen bevorzugt beispielsweise sind es Methyl, Äthyl, Propyl, Isopropyl.

Unter dem niederen Alkylrest $R_5$, der gegebenenfalls hydroxy- oder methoxy-substituiert sein kann, sind insbesondere Alkylreste mit 1—3 C-Atomen, beispielsweise der Methyl- der Äthyl-, der Hydroxyäthyl- oder der Dihydroxypropylrest zu verstehen.

Bedeutet Z einen geradkettigen oder verzweigten niederen Hydroxyalkylaminorest, so kann dieser 2—5 Kohlenstoffatome im Alkylrest enthalten. Ist Z geradkettig, so enthält der Alkylrest vorzugsweise 2—3 C-Atome, ist Z verzweigtkettig, so enthält der Alkylrest vorzugsweise 3—5 Kohlenstoffatome. Die Hydroxygruppe in Z kann als primäre oder sekundäre Hydroxygruppe vorliegen.

Als Reste Z seien beispielsweise genannt: 2-Hydroxypropylamino-, 3-Hydroxypropylamino-, 2-Hydroxy-1,1-dimethyläthylamino-, 3-Hydroxy-1,1-dimethylpropylamino- und bevorzugt der 2-Hydroxyäthylaminorest.

Der Aminosäurerest A der Formel IV kann beliebige Konfiguration haben.

Als Aminocarbonsäuren seien beispielsweise genannt: Glycin, Alanin, Valin, Serin, O-Methylserin, Prolin, Hydroxyprolin, Leucin, Isoleucin, Sarcosin, $\beta$-Alanin. Besonders bevorzugt sind $\alpha$-Aminocarbonsäuren, worin $R_7$ ein Wasserstoffatom oder eine Methylgruppe und $R_8$ ein Wasserstoffatom, eine niedere Alkylgruppe mit 1—2 Kohlenstoffatomen, die hydroxy- oder methoxysubstituiert sein kann, bedeuten.

Zur Salzbildung sind die üblicherweise verwendeten physiologisch verträglichen Basen geeignet wie beispielsweise Amine wie Glucamin, Äthanolamin, Methylglucamin, basische Aminosäuren wie Lysin, Ornithin, Arginin bzw. deren Amide oder Alkylester, Metallsalze wie Na-, K-, Ca-, Mg-salze oder Mischungen derselben.

Die Veresterung der freien Carboxylgruppe kann mit niederen Alkoholen wie beispielsweise Methanol, Äthanol, Propanol, Butanol durchgeführt werden.

Die neuen schattengebenden Verbindungen sind insbesondere zur Urographie, Angiographie, Bronchographie, zur Darstellung von Körperhöhlen sowie zur Computer-Tomographie geeignet.

Gegenüber bisher bekannten ionischen Röntgenkontrastmitteln wie beispielsweise dem Iothalamat, weisen die erfindungsgemäßen Verbindungen einen niedrigeren osmotischen Druck auf. Dies ist im Hinblick auf die im allgemeinen erforderliche Anwendung der Röntgenkontrastmittel in hochkonzentrierter Lösung besonders vorteilhaft. Gleichzeitig zeichnen sich die vorliegenden Verbindungen durch eine grosse Hydrophilie und durch die Stabilität der wässrigen Lösungen aus.

Die neuen Verbindungen der allgemeinen Formel I sind auch den in der DOS 2 523 567 beanspruchten Verbindungen aufgrund ihrer hervorragenden Eigenschaften pharmakologisch und toxikologisch überlegen, wie der Tabelle 1 am Beispiel von 5 - (3 - N - Methyl - methoxyacetamido - 5 - methylcarbamoyl - 2,4,6 - trijod - benzamido - acetamido) - N - (2 - hydroxyäthyl) - 2,4,6 - trijod - isophthalamsäure (A) zu entnehmen ist.

TABELLE 1

| Verbindung | Verträglichkeit mg J/kg Ratte<br>$LD_{50}$ i.v.<br>(7 Tage) | <br>$ED_{50}$ pericerebral<br>(24 h) | Proteinbindung | Verteilungskoeffizient |
|---|---|---|---|---|
| A | ~10 000 | ~100 | 2,5 | <0,001 |
| B | ~ 7 500 | ~ 40 | 6,8 | 0,033 |
| C | ~ 7 500 | 60 | 7,6 | 0,017 |

Die Verbindungen A, B und C wurden als Megluminsalze getestet.

*B* = 5 - (3 - N - Methyl - acetamido - 2,4,6 - trijod - 5 - methylcarbamoyl - benzamido - acetamido) - N - (2 - hydroxyäthyl) - 2,4,6 - trijod - isophthalamsäure (Ioxaglat).

*C* = 5-Acetamido-2,4,6-trijod-N-methyl-isophthalamsäure.

Die Tabelle 1 zeigt, dass die neuen Verbindungen gegenüber den Vergleichssubstanzen eine deutlich bessere allgemeine und neurale Verträglichkeit aufweisen. Ebenso ist ersichtlich, dass die Interaktion mit Proteinen sehr viel geringer ist als bei den bekannten Vergleichssubstanzen B und C. Die ausserordentliche Hydrophilie der neuen Verbindungen beweist der niedrige Verteilungskoeffizient, der in

dem System 1-Butanol/Tris-HCl-Puffer bei pH 7,6 gemessen wurde.

Die Herstellung der neuen Röntgenkontrastmittel auf Basis der Verbindungen der allgemeinen Formel I ist dadurch gekennzeichnet, dass man die schattengebende Substanz mit den in der Galenik üblichen Zusätzen in eine geeignete Applikationsform bringt. Die Konzentration der neuen Röntgenkontrastmittel im wässrigen Medium richtet sich ganz nach der röntgendiagnostischen Methode. Die bevorzugten Konzentrationen und Dosierungen der neuen Verbindungen bewegen sich in den Bereichen 50—400 mg J/ml für die Konzentration und 10—500 ml für die Dosierung. Besonders bevorzugt sind Konzentrationen zwischen 100—350 mg J/ml.

Die Art der Applikation richtet sich nach dem sichtbar zu machenden Organ.

Das Verfahren zur Herstellung der neuen Verbindungen ist dadurch gekennzeichnet, dass man ein Amin der allgemeinen Formel II

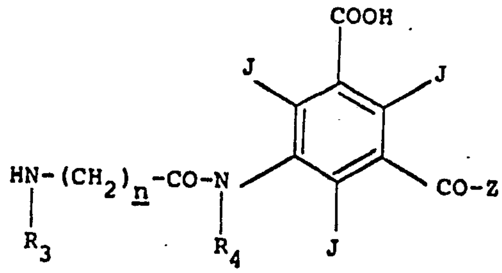

(II),

worin

$R_3$, $R_4$, Z und n die obige Bedeutung haben, mit einem reaktiven funktionellen carbonsäurederivat der allgemeinen Formel III

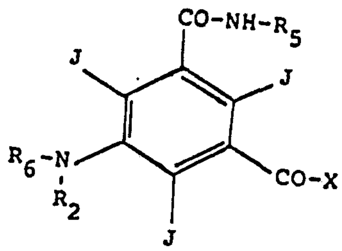

(III),

worin

$R_2$ und $R_5$ die obige Bedeutung haben, —CO—X ein reaktives carboxylgruppen- oder carbonsäureestergruppenderivat darstellt, in welchen X ein reaktiver Rest ist, und $R_6$ ein Wasserstoffatom oder ein —CO—$R_1$-Rest, wobei $R_1$ die obige Bedeutung hat, ist, umsetzt und gegebenenfalls die aromatische Aminogruppe acyliert und/oder eine Aminogruppe alkyliert und/oder die physiologisch verträglichen Salze oder niederen Alkylester bildet.

Als geeignete reaktive Säurederivate kommen in Betracht: deren Anhydride mit organischen und anorganischen Säuren wie beispielsweise mit Halogenwasserstoffsäuren, Stickstoffwasserstoffsäuren, Kohlensäurehalbestern oder deren reaktive Ester wie zum Beispiel deren leicht zugänglichen Alkyl-, Aryl- oder Cyanmethylester.

Der reaktive Rest X in der Formel (III) bedeutet demnach einen Säurerest wie zum Beispiel -Cl, -Br, -J, den Azidrest oder einen Alkoxycarbonyloxyrest oder den Rest einer reaktiven Estergruppe wie zum Beispiel —O-Alkyl, —O-Aryl oder —O—$CH_2$—C≡N.

Als bevorzugte reaktive Säurereste sind Säurehalogenide, vorzugsweise Säurechloride, zu betrachten.

Die Amidierungsreaktion wird vorzugsweise in einem polaren Lösungsmittel bei 0—100°C, bevorzugt bei 20—80°C, durchgeführt. Als Lösungsmittel kommen beispielsweise Wasser, Dioxan, Tetrahydrofuran, Methylenchlorid, Trichloräthylen, Dimethylformamid, Dimethylacetamid und deren Gemische in Betracht. Vorteilhaft verwendet man zur Neutralisation des bei der Reaktion entstehenden Chlorwasserstoffs tertiäre Amine, wie zum Beispiel Triäthylamin, Tributylamin oder Pyridin oder Alkali- oder Erdalkalihydroxide oder -carbonate wie zum Beispiel KOH, NaOH, $NaHCO_3$, $Na_2CO_3$, $Mg(OH)_2$.

Die Reaktionszeit kann zwischen 2 Stunden und etwa 2 Tagen liegen. Die Herstellung der physiologisch verträglichen Salze sowie die Veresterung der freien Carbonylgruppe mit niederen Alkoholen erfolgt nach bekannten Methoden.

Verbindungen der Formel I mit $R_3$ in der Bedeutung einer niederen Alkylgruppe können auch mit den üblichen Alkylierungsmethoden aus Verbindungen der Formel II, bei denen $R_3$ ein Wasserstoffatom bedeutet, hergestellt werden.

Wird eine Verbindung der Formel II mit einer Verbindung der Formel III, bei der $R_6$ ein Wasserstoffatom bedeutet, acyliert, so wird durch anschliessende Acylierung mit einem geeigneten reaktiven Säurederivat $R_1$—CO—X, worin X die obige Bedeutung hat, nach den üblichen Methoden eine Verbindung der allgemeinen Formel I erhalten.

Die Amine der Formel II können nach den dem Fachmann bekannten Verfahren hergestellt werden. Beispielsweise können 5-Amino-2,4,6-trijod-isophthalamsäurederivate mit einem Halogenacylhalogenid umgesetzt werden zu 5-Halogen-acylamido-2,4,6-trijod-isophthalamsäurederivate und diese werden mit Ammoniak oder einem Amin in eine Verbindung der Formel II überführt. Bedeutet $R_4$ einen niederen Alkylrest, so kann vor der Überführung in das Amin die kernständige Aminogruppe nach bekannten Verfahren alkyliert werden.

Beispiel 1

5 - [3 - (N - Methyl - methoxyacetamido) - 5 - methylcarbamoyl - 2,4,6 - trijod - benzamido - acetamido] - N - (2 - hydroxyäthyl) - 2,4,6 - trijod - isophthalamsäure

A. Herstellung der Amino-Ausgangssubstanz 5-Aminoacetamido - N - (2 - hydroxyäthyl) - 2,4,6 - trijod - isophthalamsäure

a) 3-Chloracetamido-N-(2-chloracetoxyäthyl)-2,4,6-trijod-isophthalamsäure

Zu 180,6 g 5-Amino-N-(2-hydroxyäthyl)-2,4,6-trijod-isophthalamsäure in 300 ml Dimethylacetamid werden unter Eiskühlung bei einer Maximaltemperatur von 15°C innerhalb von 45 Minuten 59,7 ml Chloracetylchlorid unter Rühren zugetropft und über Nacht verrührt. Danach werden 50 ml Wasser hinzugefügt, 30 Minuten weitergerührt, im Vakuum eingeengt, der verbleibende Sirup mit 3 Liter Wasser über Nacht gerührt, das Präparat abgesaugt, fein zermörsert und nach mehrstündigem Behandeln mit 1,5 Liter Wasser im Vakuum bei 60°C 16 Stunden getrocknet.

Ausbeute: 190,2 g (84%) vom Schmelzpunkt 239—241°C.

Analyse: $C_{14}H_{11}Cl_2J_3N_2O_6$ (754,9)

| | | |
|---|---|---|
| Berechnet: | Cl 9,39 | J 50,44 |
| Gefunden: | 9,32 | 50,79 |

b) 94.4 g der voranstehenden Bis-Chloracetyl-Verbindung werden mit 625 ml Wasser und 1 Liter konzentrierter Ammoniaklösung 5 Tage bei Raumtemperatur behandelt. Danach wird im Vakuum eingeengt, mehrmals mit Wasser nachdestilliert, in 875 ml Wasser über Nacht verrührt, der Niederschlag abgesaugt, salzfrei gewaschen und im vakuum bei 60°C getrocknet.

Ausbeute: 66,9 g (81%) vom Schmelzpunkt 255—257°C (unter Zersetzung).

Analyse: $C_{12}H_{12}J_3N_3O_5$ (659,0)

| | | | | |
|---|---|---|---|---|
| Berechnet: | C 21,87 | H 1,83 | J 57,78 | N 6,38 |
| Gefunden: | 21,92 | 2,08 | 57,75 | 6,37 |

B. Herstellung der Säurechlorid-Ausgangskomponente 2,4,6-Trijod-3-(N-methyl-methoxyacetamido)-5-methylcarbamoylbenzoesäurechlorid

a) 2,4,6-Trijod-3-(N-methyl-methoxyacetamido)-5-methylcarbamoylbenzoesäure

Zu 193,2 g 2,4,6-Trijod-3-methoxyacetamido-5-methylcarbamoylbenzoesäure in 138 ml 5 n Natronlauge werden unter starkem Rühren bei höchstens 30°C 36,9 ml Dimethylsulfat zugetropft und über Nacht verrührt. Danach wird mit 2 n Salzsäure angesäuert, der Niederschlag abgesaugt, mit 600 ml Wasser auf dem Dampfbad eine halbe Stunde behandelt, wiederum abgesaugt, mit Wasser salzfrei gewaschen und im Vakuum bei 20°C getrocknet.

Rohausbeute: 173.3 g.

Das Rohprodukt wird in 660 ml Äthylalkohol unter Zusatz von 36,4 ml 40%iger Methylaminlösung gelöst, 2 Tage bei Raumtemperatur verrührt, der ausgefallene Niederschlag abgesaugt, mit Äthylalkohol nachgewaschen und bei 60°C im Vakuum, getrocknet. Danach wird das Salz in 400 ml Wasser gelöst, mit Aktivkohle behandelt, mit konzentrierter Salzsäure angesäuert, die freie Säure nach Verrühren über Nacht, abgesaugt, mit Wasser salzfrei gewaschen und im Vakuum bei 70°C getrocknet.

Ausbeute: 128,3 g (65%) an reinem Produkt vom Schmelzpunkt bei 272—275°C (unter Zersetzung).

Analyse: $C_{13}H_{13}J_3N_2O_5$ (658,0)

| | | | | | |
|---|---|---|---|---|---|
| Berechnet: | C 23,73 | H 1,99 | J 57,86 | N 4,26 | Ä 658 |
| Gefunden: | 23,79 | 2,21 | 57,79 | 4,21 | 668 |

b) 98.7 g der voranstehenden Säure werden in 450 ml Thionylchlorid unter Zugabe von 3 ml Dimethylformamid 4 Stunden bei 65°C verrührt, über Nacht bei Raumtemperatur weitergerührt, mit 2,25 Liter absolutem Ather mehrere Stunden behandelt, abgesaugt, mit Äther nachgewaschen und bei 50°C im Vakuum getrocknet. Danach wird das Produkt in 1,1 Liter Chloroform aufgenommen, mit 200 ml gesättiger Natriumbicarbonatlösung und 3 mal mit Wasser ausgeschüttelt, die organische Phase mit Natriumsulfat getrocknet, das Lösungsmittel bei vermindertem Druck entfernt und der Rückstand bei 50°C im Vakuum getrocknet.

Ausbeute: 94,4 g (93%) vom Zersetzungspunkt bei 301 °C.
Analyse: $C_{13}H_{12}ClJ_3N_2O_4$ (676,4)

| | | | |
|---|---|---|---|
| Berechnet: | C 23,08 | H 1,79 | N 4,14 |
| Gefunden: | 22,87 | 1,88 | 4,09 |

c) Zu einer Suspension von 55,4 g 5-Aminoacetamido-N-(2-hydroxyäthyl)-2,4,6-trijod-isophthalamsäure in 80 ml Dimethylacetamid werden unter Zusatz von 25,5 ml Triäthylamin 54,1 g 2,4,6-Trijod-3-(N-methylmethoxyacetamido)-5-methylcarbamoyl-benzoesäurechlorid zugegeben, 3 Stunden bei 50°C und dann über Nacht bei Raumtemperatur verrührt, gegebenenfalls von wenig Ungelöstem abfiltriert, mit 1 Liter Wasser verdünnt, die leicht trübe Lösung mit Aktivkohle behandelt und nach Entfernen der Kohle mit 50 ml konzentrierter Salzsäure angesäuert. Danach wird über Nacht verrührt, vom Niederschlag abgetrennt, mit etwas Wasser gewaschen und im Vakuum bei 60°C getrocknet. Nach 16-stündigem Verrühren des trockenen Präparats mit 700 ml Wasser wird abgesaugt, mit Wasser salzfrei gewaschen und bei 60°C im Vakuum getrocknet.
Ausbeute: 68,8 g (66%) vom Schmelzpunkt 292° (unter Zersetzung)
Analyse: $C_{25}H_{23}J_6N_5O_9$ (1298,9)

| | | | | |
|---|---|---|---|---|
| Berechnet: | C 23,12 | H 1,78 | J 58,62 | Ä 1299 |
| Gefunden: | 23,11 | 1,77 | 58,53 | 1289 |

Beispiel 2

5 - [3 - (N - Methyl - acetamido) - 5 - methylcarbamoyl - 2,4,6 - trijod - benzamido - acetamido] - N - (2 - hydroxy - 1 - methylcarbamoyl - äthyl) - 2,4,6 - trijod - isophthalamsäure

*Herstellung der Aminoausgangskomponente*

A. 5-Aminoacetamido-N-(2-hydroxy-1-methylcarbamoyläthyl)-2,4,6-trijod-isophthalamsäure

a) Zu 98,9 g 5-Amino-N-(2-hydroxy-1-methylcarbamoyläthyl)-2,4,6-trijod-isophthalamsäure in 150 ml Dimethylformamid werden unter Eiskühlung und Rühren innerhalb von 40 Minuten 35,8 ml Chloracetylchlorid bei höchstens 15°C zugetropft, eine Stunde im Eisbad und danach über Nacht bei Raumtemperatur verrührt. Die Lösung wird dann in 2 Liter Wasser unter Rühren eingetropft, 16 Stunden weiterverrührt, der Niederschlag abgetrennt, mit Wasser einige Stunden behandelt, abgesaugt, mit Wasser salzfrei gewaschen und im Vakuum getrocknet.

b) Die erhaltene 103,5 g (85%) Bis-N,O-Chloracetylverbindung vom Schmelzpunkt 190—193°C wird in 1020 ml konzentriertem Ammoniak unter Zusatz von 640 ml Wasser 8 Tage lang unter Lichtausschluss stehengelassen, im Vakuum eingeengt, mehrmals mit Wasser nachdestilliert, der Rückstand eine Stunde auf dem Dampfbad in 500 ml Wasser erhitzt, über Nacht bei Raumtemperatur verrührt, das Präparat abgesaugt, mit Wasser nachgewaschen und im Vakuum bei 60°C getrocknet.
Ausbeute: 45,6 g (50%) vom Schmelzpunkt 255—257°C (unter Zersetzung).
Analyse: $C_{14}H_{15}J_3N_4O_6$ (716,0)

| | | | | | |
|---|---|---|---|---|---|
| Berechnet: | C 23,48 | H 2,11 | J 53,17 | N 7,83 | Ä 716 |
| Gefunden: | 23,49 | 2,08 | 53,14 | 7,85 | 720 |

B. Zu 35,8 g der voranstehenden Aminoverbindung in 50 ml Dimethylacetamid werden 32,3 g 2,4,6-Trijod-3-(N-methylacetamido)-5-methylcarbamoyl-benzoesäurechlorid und 15,9 ml Triäthylamin zugefügt, 3 Stunden bei 50°C und dann über Nacht bei Raumtemperatur verrührt. Die Reaktionslösung wird nachfolgend in 650 ml Wasser aufgenommen, mit 10 g Aktivkohle eine Stunde behandelt, das Filtrat nach Entfernen der Kohle mit 50 ml konzentrierter Salzsäure angesäuert und über Nacht bei Raumtemperatur verrührt. Danach wird der Niederschlag abgesaugt, mit etwas Wasser nachgewaschen, im Vakuum bei 50°C getrocknet, das trockene Produkt mit frischem Wasser verrührt, abgesaugt, mit Wasser gewaschen und im Vakuum bei 60°C getrocknet.
Ausbeute: 33,7 g (51%) vom Schmelzpunkt ~276°C (unter Zersetzung).
Analyse: $C_{26}H_{24}J_6N_6O_9$ (1325,9)

| | | | | | |
|---|---|---|---|---|---|
| Berechnet: | C 23,55 | H 1,82 | J 57,43 | N 6,34 | Ä 1326 |
| Gefunden: | 23,65 | 1,84 | 57,70 | 6,29 | 1332 |

Beispiel 3

5 - [3 - (N - Methyl - acetamido) - 5 - methylcarbamoyl - 2,4,6 - trijod - benzamidoacetamido] - 2,4,6 - trijod - N - (methylcarbamoylmethyl) - isophthalamsäure

A. *Herstellung der Aminoausgangskomponente*

5-Aminoacetamido-2,4,6-trijod-N-methylcarbamoylmethyl-isophthalamsäure

a) 3-Chloracetamido-2,4,6-trijod-N-methylcarbamoylmethyl-isophthalamsäure

Die Darstellung erfolgt analog Beispiel 1 A.a. aus 78,6 g 3-Amino-2,4,6-trijod-N-methylcarbamoylmethyl-isophthalamsäure und 29,8 ml Chloracetylchlorid.
Ausbeute: 79,5 g (90%) vom Schmelzpunkt 290—291°C (unter Zersetzung).
Analyse: $C_{13}H_{11}ClJ_3N_3O_5$ (705,4)

| | | | | | |
|---|---|---|---|---|---|
| Berechnet: | C 22,13 | H 1,57 | Cl 5,03 | J 53,97 | N 5,96 |
| Gefunden: | 22,19 | 1,50 | 5,32 | 53,48 | 5,97 |

b) Durch achttägiges Behandeln von 70,5 g des voranstehenden Chloracetylpräparates mit 800 ml konzentriertem Ammoniak unter Zusatz von 500 ml Wasser wird, wie in Beispiel 1 A.b. beschrieben, die entsprechende Aminoverbindung erhalten.
Ausbeute: 55,9 g (82%) vom Schmelzpunkt 258—259°C (unter Zersetzung).
Analyse: $C_{13}H_{13}I_3N_4O_5$ (686,0)

| | | | | |
|---|---|---|---|---|
| Berechnet: | C 22,76 | H 1,91 | J 55,50 | N 8,17 |
| Gefunden: | 22,50 | 2,13 | 55,60 | 8,20 |

c) Analog Beispiel 2 B wird aus der voranstehenden Aminoverbindung und 2,4,6-Trijod-3-(N-methyl-acetamido)-5-methylcarbamoyl-benzoesäurechlorid in Dimethylacetamid die Titelverbindung hergestellt.
Schmelzpunkt: 272°C (unter Zersetzung).
Analyse: $C_{25}H_{22}I_6N_6O_8$ (1295,9)

| | | | | | |
|---|---|---|---|---|---|
| Berechnet: | C 23,17 | H 1,71 | J 58,76 | N 6,49 | Ä 1296 |
| Gefunden: | 23,22 | 1,66 | 58,40 | 6,39 | 1288 |

Beispiel 4

5 - (3 - Acetamido - 5 - methylcarbamoyl - 2,4,6 - trijod - benzamidoacetamido) - N - (2 - hydroxy - 1 - methylcarbamoyläthyl) - 2,4,6 - trijodisophthalamsäure

A 5 - (3 - Amino - 5 - methylcarbamoyl - 2,4,6 - trijod - benzamidoacetamido) - N - (2 - hydroxy - 1 - methylcarbamoyläthyl) - 2,4,6 - trijod - isophthalamsäure

Zu 35,8 g 5 - Aminoacetamido - N - (2 - hydroxy - 1 - methylcarbamoyläthyl) - 2,4,6 - trijod - isophthalamsäure (Beispiel 2 A.) in 50 ml Dimethylacetamid und 15,9 ml Triäthylamin werden 29,5 g 3 - Amino - 2,4,6 - trijod - 5 - methylcarbamoyl - benzoesäurechlorid zugefügt, 3 Stunden bei 50°C und danach bei Raumtemperatur verrührt. Dann werden 650 ml Wasser hinzugefügt, die Lösung mit Aktivkohle behandelt, nach Entfernen der Kohle mit 50 ml konzentrierter Salzsäure angesäuert, der Niederschlag abgesaugt, mit 500 ml Wasser ausgerührt und nach erneutem Absaugen und Waschen mit Wasser im Vakuum bei 60°C getrocknet.
Ausbeute: 32,6 (51%) vom Schmelzpunkt 253—255°C (unter Zersetzung).
Analyse: $C_{23}H_{20}J_6N_6O_8$ (1269,9)

| | | | | | |
|---|---|---|---|---|---|
| Berechnet: | C 21,75 | H 1,59 | J 59,96 | N 6,62 | Ä 1270 |
| Gefunden: | 21,91 | 1,68 | 60,29 | 6,65 | 1260 |

B. Zu 19,0 g der voranstehenden Aminoverbindung in 36 ml Dimethylacetamid werden bei 0°C unter Rühren 3,6 ml Acetylchlorid zugetropft, über Nacht bei Raumtemperatur und Dann nach Zugabe von etwas Wasser weitere 30 Minuten verrührt. Nach Einengen im Vakuum wird der Rückstand mit 200 ml Wasser verrührt, der Niederschlag abgesaugt, dieser wird dann in 50 ml 2 n Natronlauge und 150 ml Wasser gelöst, die Lösung wird mit Aktivkohle behandelt, das Filtrat nach Entfernen der Kohle mit 20 ml konzentrierter Salzsäure angesäuert und über Nacht bei Raumtemperatur verrührt. Das Präparat wird nachfolgend isoliert, in 100 ml Wasser zum Sieden erhitzt, nach Abkühlen abgesaugt, salzfrei gewaschen und im Vakuum bei 70°C getrocknet.
Ausbeute: 12,4 g (63%) vom Schmelzpunkt ~263°C (unter Zersetzung).
Analyse: $C_{25}H_{22}J_6N_6O_9$ (1311,9)

| | | | | |
|---|---|---|---|---|
| Berechnet: | C 22,89 | H 1,69 | J 58,04 | N 6,41 |
| Gefunden: | 22,90 | 1,70 | 57,74 | 6,27 |

Beispiel 5

5 - [3 - (N - Methyl - acetamido) - 5 - methylcarbamoyl - 2,4,6 - trijod - N - methyl-benzamido - acetamido] - N - (2 - hydroxyäthyl) - 2,4,6 - trijod - isophthalamsäure

A. *Herstellung der Aminoausgangskomponente*

5-Methylaminoacetamido-N-(2-hydroxyäthyl)-2,4,6-trijod-isophthalamsäure

Darstellung analog der Aminoverbindung in Beispiel 1 A. aus 56,6 g 3-Chloracetamido-N-(2-chloracetoxyäthyl)-2,4,6-trijod-isophthalamsäure (Beispiel 1A.a) und 600 ml 40%igem wässrigem Methylamin unter Zusatz von 375 ml Wasser.
Ausbeute: 42,6 g (84%) vom Schmelzpunkt 247—249°C (unter Zersetzung).

Analyse: $C_{13}H_{14}J_3N_3O_5$ (673,0)

| | | | | | |
|---|---|---|---|---|---|
| Berechnet: | C 23,20 | H 2,10 | J 56,57 | N 6,24 | Ä 673 |
| Gefunden: | 23,38 | 2,10 | 56,62 | 6,13 | 674 |

B. *Herstellung der Titelverbindung*

Durch Umsetzung von 33,7 g der voranstehenden Verbindung mit 32,3 g 2,4,6-Trijod-3-(N-methyl-acetamido)-5-methylcarbamoyl-benzoesäurechlorid in 100 ml Dimethylformamid wird die Titelverbindung, wie in Beispiel 2 beschrieben, hergestellt.
Ausbeute: 46 g (72%) vom Schmelzpunkt ca. 310°C (unter Zersetzung).
Analyse: $C_{25}H_{23}J_6N_5O_8$ (1282,9)

| | | | | | |
|---|---|---|---|---|---|
| Berechnet: | C 23,40 | H 1,81 | J 59,35 | N 5,46 | Ä 1283 |
| Gefunden: | 23,72 | 1,97 | 59,10 | 5,60 | 1289 |

Beispiel 6

*Herstellung einer gebrauchsfertigen Methylglucaminsalzlösung*

| | |
|---|---|
| 5 - [3 - (N - Methyl - methoxyacetamido) - 5 - methylcarbamoyl - 2,4,6 - trijod - benzamidoacetamido] - N - (2 - hydroxyäthyl) - 2,4,6 - trijod - isophthalamsäure | 648,2 g |
| N-Methylglucamin | 97,3 g |
| Calcium-dinatriumedetat | 0,1 g |
| Bidestilliertes Wasser | ad 1000,0 ml |

Die Lösung wird in Ampullen oder Multivials abgefüllt und bei 120°C sterilisiert. Sie enthält 380 mg J/ml.

Beispiel 7

*Herstellung einer gebrauchsfertigen Methylglucaminsalzlösung*

| | |
|---|---|
| 5 - [3 - (N - Methyl - acetamido) - 5 - methylcarbamoyl - 2,4,6 - trijod - benzamidoacetamido] - N - (2 - hydroxy - 1 - methylcarbamoyläthyl) - 2,4,6 - trijod - isophthalamsäure | 522,4 g |
| N-Methylglucamin | 76,8 g |
| Calcium-dinatriumedetat | 0,1 g |
| Bidestilliertes Wasser | ad 1000,0 ml |

Die Lösung wird in Ampullen oder Multivials abgefüllt und bei 120°C sterilisiert. Sie enthält 300 mg J/ml.

**Patentansprüche für die Vertragsstaaten: BE, CH, DE, FR, GB, IT, LU, NL, SE**

1. Verbindungen der allgemeinen Formel I

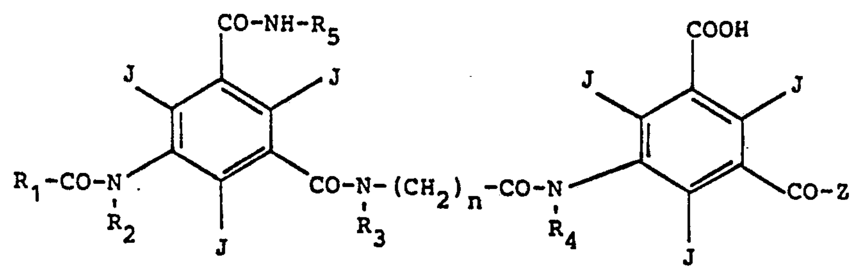

(I),

worin

$R_1$ einen gegebenenfalls mit einer Methoxy- oder Ethoxygruppe substituierten Alkylrest mit 1 bis 4 Kohlenstoffatomen darstellt,

$R_2$, $R_3$ und $R_4$ ein Wasserstoffatom oder einen niederen Alkylrest mit 1 bis 4 Kohlenstoffatomen bedeuten,

$R_5$ einen gegebenenfalls hydroxy- oder methoxysubstituierten niederen Alkylrest darstellt,

n die Zahlen 1, 2 oder 3 bedeuten und

Z einen —A—$NHCH_3$-Rest darstellt, worin A ein Aminosäurerest der allgemeinen Formel IV

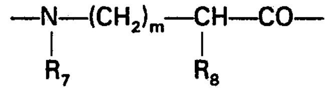

(IV),

ist, der mit seiner CO-Gruppe an —$NHCH_3$ und mit seiner Aminogruppe an CO gebunden ist und in dem m = 0 oder 1, $R_7$ ein Wasserstoffatom oder eine niedere Alkylgruppe mit 1—4 Kohlenstoffatomen, vorzugsweise 1—2 C-Atomen, $R_8$ ein Wasserstoffatom oder eine niedere Alkylgruppe mit 1—4 C-Atomen, die gerade oder verzweigtkettig sein kann und durch Hydroxy- oder niedere Alkoxygruppen mit 1—2 C-Atomen substituiert sein kann, und $R_7$ und $R_8$ auch gemeinsam eine ringbildende Propylen- oder Hydroxypropylengruppe bedeuten, oder

Z einen geradkettigen oder verzweigten niederen Hydroxyalkylaminorest darstellt, wenn R einen monomethoxy- oder monoethoxysubstituierten Alkylrest mit 1 bis 4 Kohlenstoffatomen und/oder $R_3$ einen niederen Alkylrest mit 1 bis 4 Kohlenstoffatomen bedeutet, sowie deren niedere Alkylester und deren Salze mit physiologisch verträglichen Basen.

2. 5 - [3 - (N - Methyl - methoxyacetamido) - 5 - methylcarbamoyl - 2,4,6 - trijod - benzamido - acetamido] - N - (2 - hydroxyäthyl) - 2,4,6 - trijod - isophthalamsäure gemäß Anspruch 1.

3. 5 - [3 - (N - Methyl - acetamido) - 5 - methylcarbamoyl - 2,4,6 - trijod - benzamido - acetamido] - N - (2 - hydroxy - 1 - methylcarbamoyl - äthyl) - 2,4,6 - trijod - isophthalamsäure gemäß Anspruch 1.

4. 5 - [3 - (N - Methyl - acetamido) - 5 - methylcarbamoyl - 2,4,6 - trijod - benzamido - acetamido] - 2,4,6 - trijod - N - (methylcarbamoylmethyl) - isophthalamsäure gemäß Anspruch 1.

5. 5 - (3 - Acetamido - 5 - methylcarbamoyl - 2,4,6 - trijod - benzamido - acetamido) - N - (2 - hydroxy - 1 - methylcarbamoyläthyl) - 2,4,6 - trijod - isophthalamsäure gemäß Anspruch 1.

6. 5 - [3 - (N - Methyl - acetamido) - 5 - methylcarbamoyl - 2,4,6 - trijod - N - methylbenz - amido acetamido] - N - (2 - hydroxyäthyl) - 2,4,6 - trijod - isophthalamsäure gemäß Anspruch 1.

7. Verfahren zur Herstellung von Verbindung der allgemeinen Formel I gemäss Anspruch 1, dadurch gekennzeichnet, dass man ein Amin der allgemeinen Formel II

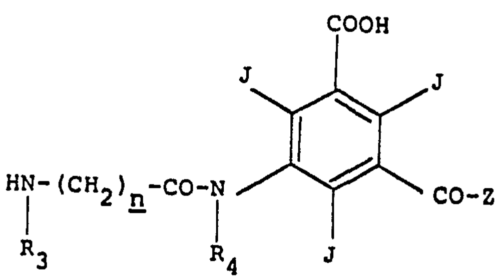

(II),

worin

$R_3$, $R_4$, Z und n die in Anspruch 1 angegebene Bedeutung haben, mit einem reaktiven funktionellen Carbonsäurederivat der allgemeinen Formel III

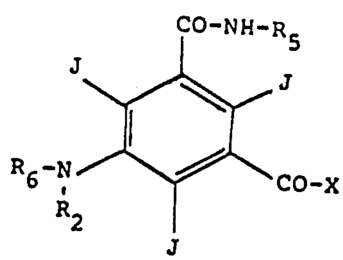

(III),

worin

$R_2$ und $R_5$ die in Anspruch 1 angegebene bedeutung haben, —CO—X ein reaktives Carboxylgruppen- oder Carbonsäureestergruppenderivat darstellt, in welchem X ein reaktiver Rest ist, und $R_6$ ein Wasserstoffatom oder einen —CO—$R_1$-Rest bedeutet, umsetzt und im Falle von $R_6$= Wasserstoff die aromatische Aminogruppe mit einem reaktiven Säurederivat $R_1$—CO—X, wobei X ein reaktiver Rest ist und $R_1$ die in Anspruch 1 angegebene Bedeutung hat, acyliert und/oder eine Aminogruppe alkyliert und/oder die physiologisch verträglichen Salze mit Basen oder die niederen Alkylester bildet.

8. Röntgenkontrastmittel, dadurch gekennzeichnet, daß es mindestens eine Verbindung der allgemeinen Formel I gemäß Anspruch 1 enthält.

**Patentanspruch für den Vertragsstaat: AT**

1. Verfahren zur Herstellung von Verbindungen der allgemeinen Formel I

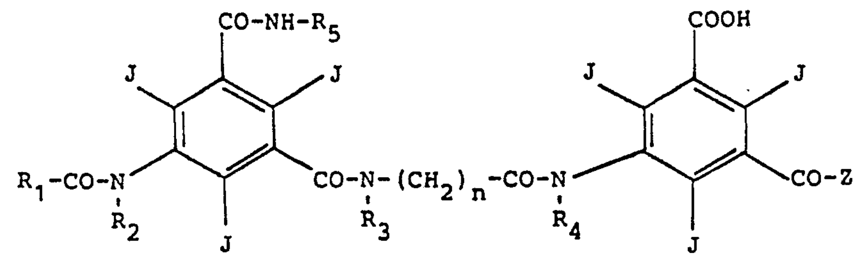

(I),

worin

$R_1$ einen gegebenenfalls mit einer Methoxy- oder Ethoxygruppe substituierten Alkylrest mit 1 bis 4 Kohlenstoffatomen darstellt,

$R_2$, $R_3$ und $R_4$ ein Wasserstoffatom oder einen niederen Alkylrest mit 1 bis 4 Kohlenstoffatomen bedeuten,

$R_5$ einen gegebenenfalls hydroxy- oder methoxysubstituierten niederen Alkylrest darstellt,

n die Zahlen 1, 2 oder 3 bedeuten und

Z einen —A—$NHCH_3$-Rest darstellt, worin A ein Aminosäurerest der allgemeinen Formel IV

$$-\underset{\substack{|\\R_7}}{N}-(CH_2)_m-\underset{\substack{|\\R_8}}{CH}-CO- \qquad (IV),$$

ist, der mit seiner CO-Gruppe an —$NHCH_3$ und mit seiner Aminogruppe an CO gebunden ist und in dem m = 0 oder 1, $R_7$ ein Wasserstoffatom oder eine niedere Alkylgruppe mit 1—4 Kohlenstoffatomen, vorzugsweise 1—2 C-Atomen, $R_8$ ein Wasserstoffatom oder eine niedere Alkylgruppe mit 1—4 C-Atomen, die gerade- oder verzweigtkettig sein kann und durch Hydroxy- oder niedere Alkoxygruppen mit 1—2 C-Atomen substituiert sein kann, und $R_7$ und $R_8$ auch gemeinsam eine ringbildende Propylen- oder Hydroxypropylengruppe bedeuten, oder

Z einen geradkettigen oder verzweigten niederen Hydroxyalkylaminorest darstellt, wenn $R_1$ einen monomethoxy- oder monoethoxysubstituierten Alkylrest mit 1—4 Kohlenstoffatomen und/oder $R_3$ einen niederen Alkylrest mit 1 bis 4 Kohlenstoffatomen bedeutet, sowie deren niedere Alkylester und deren Salze mit physiologisch verträglichen Basen, dadurch gekennzeichnet, dass man ein Amin der allgemeinen Formel II

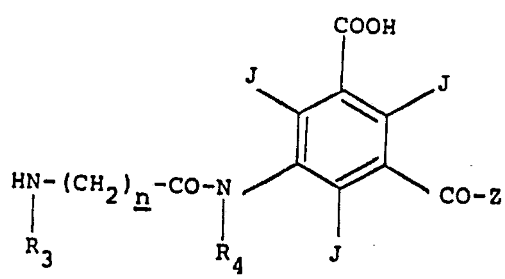

(II),

worin

$R_3$, $R_4$, Z und n die vorstehend angegebene Bedeutung haben, mit einem reaktiven funktionellen Carbonsäurederivat der allgemeinen Formel III

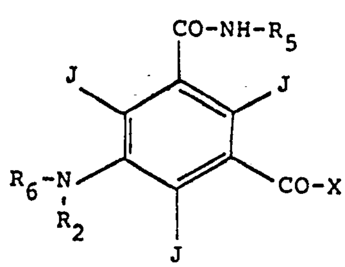

(III),

worin

$R_2$ und $R_5$ die vorstehend angegebene Bedeutung haben, —CO—X ein reaktives Carbonylgruppen- oder

Carbonsäureestergruppenderivat darstellt, in welchem X ein reaktiver Rest ist, und $R_6$ ein Wasserstoffatom oder einen CO—$R_1$-Rest bedeutet, umsetzt und im Falle von $R_6$ = Wasserstoff die aromatische Aminogruppe mit einem reaktiven Säurederivat $R_1$—CO—X, wobei X ein reaktiver Rest ist und $R_1$, die in Anspruch 1 angegebene Bedeutung hat, acyliert und/oder eine Aminogruppe alkyliert und/oder die physiologisch verträglichen Salze mit Basen oder die niederen Alkylester bildet.

**Revendications pour les Etats contractants: BE, CH, DE, FR, GB, IT, LU, NL, SE**

1. Les composés de formule générale I:

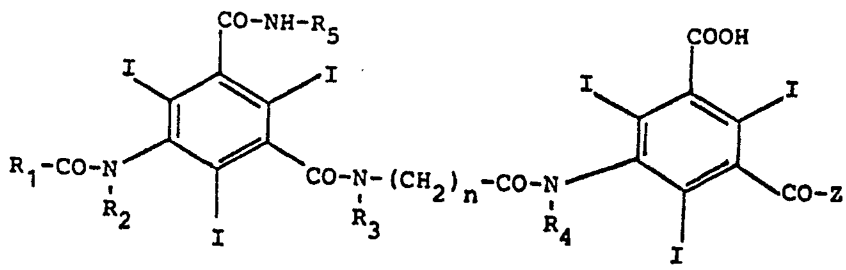

(I)

dans laquelle:

$R_1$ représente un alkyle en $C_1$ à $C_4$ éventuellement substitué par un groupe méthoxy ou éthoxy,
$R_2$, $R_3$, $R_4$ représentent chacun un atome d'hydrogène ou un alkyle en $C_1$—$C_4$,
$R_5$ représente un alkyle inférieur éventuellement substitué par un groupe méthoxy ou éthoxy,
n est le nombre 1, 2 ou 3, et
Z un radical —A—$NHCH_3$, A étant un reste d'aminoacide de formule IV:

$$-\underset{\displaystyle R_7}{\underset{|}{N}}-(CH_2)_m-\underset{\displaystyle R_8}{\underset{|}{CH}}-CO- \qquad (IV)$$

lié par son groupe CO au radical —$NHCH_3$ et par son groupe amino ou groupe CO, et dans lequel m = 0 ou 1, $R_7$ est un atome d'hydrogène ou un alkyle en $C_1$—$C_4$, de préférence en $C_1$ ou $C_2$, et $R_8$ un atome d'hydrogène ou un alkyle en $C_1$—$C_4$ à chaîne droite ou ramifiée et pouvant être substitué par des groupes hydroxy ou alcoxy en $C_1$ ou $C_2$, ou encore $R_7$ et $R_8$ forment ensemble un groupe propylène ou hydroxypropylène constituant un cycle, ou bien,

Z est un radical hydroxy-alkylamino inférieur à chaîne droite ou ramifiée si $R_1$ est un groupe monométhoxy- ou monoéthoxy-alkyle en $C_1$—$C_4$ et/ou $R_3$ un alkyle en $C_1$ à $C_4$, ainsi que leurs esters alkyliques inférieurs et leurs sels formés avec des bases physiologiquement compatibles.

2. Acide 5 - [3 - (N - méthyl - méthoxyacétamido) - 5 - méthylcarbamoyl - 2,4,6 - triiodo - benzamido - acétamido] - N - (2 - hydroxyéthyl) - 2,4,6 - triiodo - isophtalamique selon la revendication 1.

3. Acide 5 - [3 - (N - méthyl - acétamido) - 5 - méthylcarbamoyl - 2,4,6 - triiodo - benzamido - acétamido] - N - (2 - hydroxy - 1 - méthylcarbamoyl - éthyl) - 2,4,6 - triiodo - isophtalamique selon la revendication 1.

4. Acide 5 - [3 - (N - méthyl - acétamido) - 5 - méthylcarbamoyl - 2,4,6 - triiodo - benzamido - acétamido] - 2,4,6 - triiodo - N - (méthylcarbamoylméthyl) - isophtalamique selon la revendication 1.

5. Acide 5 - (3 - acétamido - 5 - méthylcarbamoyl - 2,4,6 - triiodo - benzamido - acétamido) - N - (2 - hydroxy - 1 - méthylcarbamoyléthyl) - 2,4,6 - triiodo - isophtalamique selon la revendication 1.

6. Acide 5 - [3 - (N - méthyl - acétamido) - 5 - méthylcarbamoyl - 2,4,6 - triiodo - N - méthylbenzamido - acétamido] - N - (2 - hydroxyéthyl) - 2,4,6 - triiodo - isophtalamique selon la revendication 1.

7. Procédé de préparation des composés de formule générale I selon la revendication 1, caractérisé en ce que l'on fait réagir une amine de formule générale II:

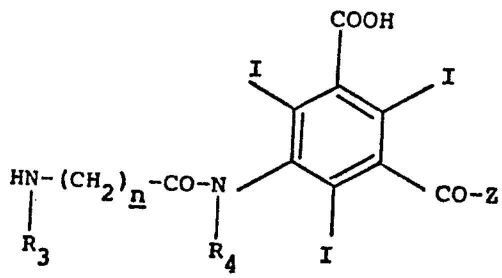

(II)

dans laquelle les symboles $R_3$, $R_4$, Z et n ont les significations données à la revendication 1, avec un dérivé fonctionnel réactif d'un acide carboxylique de formule générale III:

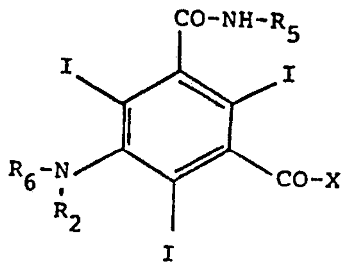

(III)

dans laquelle $R_2$ et $R_5$ ont les significations données la revendication 1, —CO—X représente un dérivé réactif de groupe carboxylique ou d'ester carboxylique, X étant un radical réactif, et $R_6$ représente un atome d'hydrogène ou un radical —CO—$R_1$, et, si $R_6$ est l'hydrogène, on acyle le groupe amino sur le cycle aromatique avec un dérivé réactif d'acide $R_1$—CO—X, et/ou on alkyle un groupe amino et/ou on forme les sels physiologiquement compatibles avec des bases ou les esters alkyliques inférieurs des composés ainsi obtenus.

8. Produits de contraste pour rayons X, caractérisés en ce qu'ils contiennent comme opacifiant un ou plusieurs composés de formule générale I selon la revendication 1.

**Revendication pour l'Etat contractant: AT**

1. Procédé de préparation des composés de formule générale I

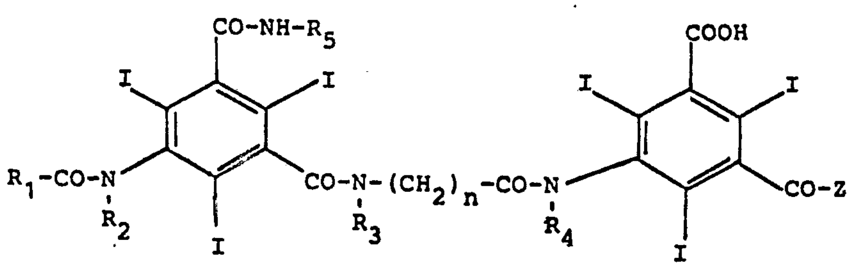

(I)

dans laquelle:

$R_1$ représente un alkyle en $C_1$ à $C_4$ éventuellement substitué par un groupe méthoxy ou éthoxy,
$R_2$, $R_3$, $R_4$ représentent chacun un atome d'hydrogène ou un alkyle en $C_1$—$C_4$,
$R_5$ représente un alkyle inférieur éventuellement substitué par un groupe méthoxy ou éthoxy,
n est le nombre 1, 2 ou 3, et
Z un radical —A—$NHCH_3$, A étant un reste d'aminoacide de formule IV:

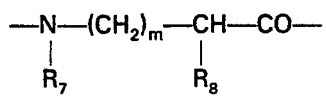

(IV)

lié par son groupe CO au radical —$NHCH_3$ et par son groupe amino ou groupe CO, et dans lequel m = 0 ou 1, $R_7$ est un atome d'hydrogène ou un alkyle en $C_1$—$C_4$, de préférence en $C_1$ ou $C_2$, et $R_8$ un atome d'hydrogène ou un alkyle en $C_1$—$C_4$ à chaîne droite ou ramifiée et pouvant être substitué par des groupes hydroxy ou alcoxy en $C_1$ ou $C_2$, ou encore $R_7$ et $R_8$ forment ensemble un groupe propylène ou

hydroxypropylène constituant un cycle, ou bien,

Z est un radical hydroxy-alkylamino inférieur à chaîne droite ou ramifiée si $R_1$ est un groupe mono-méthoxy- ou monoéthoxy-alkyle en $C_1$—$C_4$ et/ou $R_3$ un alkyle en $C_1$ à $C_4$, ainsi que leurs esters alkyliques inférieurs et leurs sels formés avec des bases physiologiquement compatibles, caractérisé en ce que l'on fait réagir une amine de formule générale II:

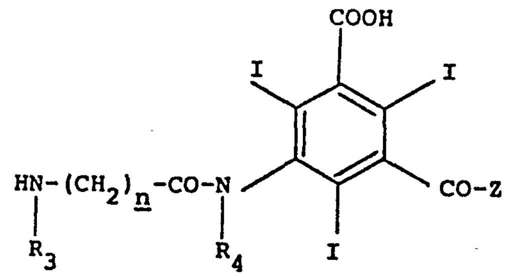

(II)

dans laquelle les symboles $R_3$, $R_4$, Z et n ont les significations données à la revendication 1, avec un dérivé fonctionnel réactif d'un acide carboxylique de formule générale III:

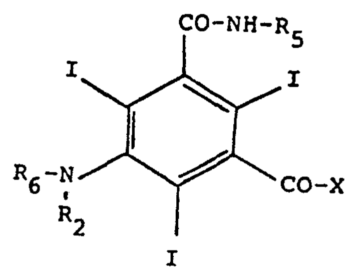

(III)

dans laquelle $R_2$ et $R_5$ ont les significations données à la revendication 1, —CO—X représente un dérivé réactif de groupe carboxylique ou d'ester carboxylique, X étant un radical réactif, et $R_6$ représente un atome d'hydrogène ou un radical —CO—$R_1$, et, si $R_6$ l'hydrogène, on acyle le groupe amino sur le cycle aromatique avec un dérivé réactif d'acide $R_1$—CO—X, et/ou on alkyle un groupe amino et/ou on forme les sels physiologiquement compatibles avec des bases ou les esters alkyliques inférieurs des composés ainsi obtenus.

**Claims for the Contracting States: BE, CH, DE, FR, GB, IT, LU, NL, SE**

1. Compounds of the general formula I

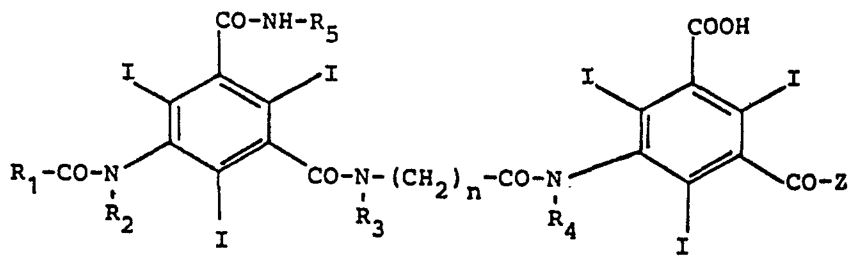

(I)

in which

$R_1$ represents an alkyl radical having from 1 to 4 carbon atoms optionally substituted by a methoxy or an ethoxy group,

$R_2$, $R_3$ and $R_4$ represent a hydrogen atom or a lower alkyl radical having from 1 to 4 carbon atoms,

$R_5$ represents a lower alkyl radical optionally substituted by hydroxy or methoxy,

n represents the numbers 1, 2 or 3, and

Z represents an —A—$NHCH_3$ radical in which A is an amino acid radical of the general formula IV

$$-\underset{R_7}{N}-(CH_2)_m-\underset{R_8}{CH}-CO- \qquad (IV)$$

which is bonded by its CO group to —$NHCH_3$ and by its amino group to CO and in which m = 0 or 1, $R_7$ represents a hydrogen atom or a lower alkyl group having from 1 to 4 carbon atoms preferably 1 or 2 carbon atoms, $R_8$ represents a hydrogen atom or a lower alkyl group having from 1 to 4 carbon atoms which may be straight-chain or branched and may be substituted by hydroxy groups or lower alkoxy groups having 1 or 2 carbon atoms, and $R_7$ and $R_8$ also together represent a ring-forming propylene group or hydroxypropylene group, or

Z represents a straight-chain or branched lower hydroxyalkylamino radical, whenever $R_1$ represents a monomethoxy- or a monoethoxy-substituted alkyl radical having from 1 to 4 carbon atoms and/or $R_3$ represents a lower alkyl radical having from 1 to 4 carbon atoms, the lower alkyl esters thereof and the salts thereof with physiologically tolerable bases.

2. 5 - [3 - (N - methylmethoxyacetamido) - 5 - methylcarbamoyl - 2,4,6 - triiodobenzamidoacetamido] - N - (2 - hydroxyethyl) - 2,4,6 - triiodoisophthalamic acid according to claim 1.

3. 5 - [3 - (N - methylacetamido) - 5 - methylcarbamoyl - 2,4,6 - triiodobenzamidoacetamido] - N - (2 - hydroxy - 1 - methylcarbamoylethyl) - 2,4,6 - triiodoisophthalamic acid according to claim 1.

4. 5 - [3 - (N - methylacetamido) - 5 - methylcarbamoyl - 2,4,6 - triiodobenzamidoacetamido] - 2,4,6 - triiodo - N - (methylcarbamoylmethyl)isophthalamic acid according to claim 1.

5. 5 - (3 - acetamido - 5 - methylcarbamoyl - 2,4,6 - triiodobenzamidoacetamido) - N - (2 - hydroxy - 1 - methylcarbamoylethyl) - 2,4,6 - triiodoisophthalamic acid according to claim 1.

6. 5 - [3 - (N - methylacetamido) - 5 - methylcarbamoyl - 2,4,6 - triiodo - N - methylbenzamido acetamido] - N - (2 - hydroxyethyl) - 2,4,6 - triiodoisophthalamic acid according to claim 1.

7. Process for the preparation of compounds of the general formula I according to claim 1, characterised in that an amine of the general formula II

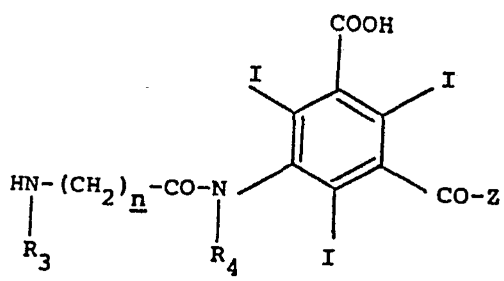

(II),

in which $R_3$, $R_4$, Z and *n* have the meanings given in claim 1, is reacted with a reactive functional carboxylic acid derivative of the general formula III

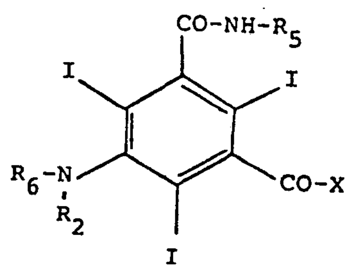

(III),

in which

$R_2$ and $R_5$ have the meanings given in claim 1, —CO—X represents a reactive carboxy group derivative or a reactive carboxylic acid ester group derivative in which X is a reactive radical, and $R_6$ represents a hydrogen atom or a —CO—$R_1$ radical and, in the case of $R_6$ = hydrogen, the aromatic amino group is acylated with a reactive acid derivative $R_1$—CO—X in which X is a reactive radical and $R_1$ has the meaning given in claim 1 and/or an amino group is alkylated and/or the physiologically tolerable salts with bases or the lower alkyl esters are formed.

8. X-ray contrast medium, characterised in that it contains at least one compound of the general formula I according to claim 1.

**Claim for the Contracting State: AT**

1. Process for the preparation of compounds of the general formula I

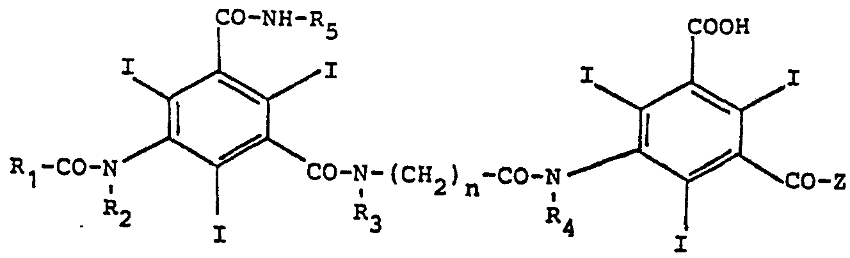

(I)

in which

$R_1$ represents an alkyl radical having from 1 to 4 carbon atoms optionally susbtituted by a methoxy or an ethoxy group,

$R_2$, $R_3$ and $R_4$ represent a hydrogen atom or a lower alkyl radical having from 1 to 4 carbon atoms,

$R_5$ represents a lower alkyl radical optionally susbtituted by hydroxy or methoxy,

n represents the numbers 1, 2 or 3, and

Z represents an —A—$NHCH_3$ radical in which A is an amino acid radical of the general formula IV

$$-\underset{R_7}{\underset{|}{N}}-(CH_2)_m-\underset{R_8}{\underset{|}{CH}}-CO- \qquad (IV)$$

which is bonded by its CO group to —$NHCH_3$ and by its amino group to CO and in which $m = 0$ or 1, $R_7$ represents a hydrogen atom or a lower alkyl group having from 1 to 4 carbon atoms preferably 1 or 2 carbon atoms, $R_8$ represents a hydrogen atom or a lower alkyl group having from 1 to 4 carbon atoms which may be straight-chain or branched and may be substituted by hydroxy groups or lower alkoxy groups having 1 or 2 carbon atoms, and $R_7$ and $R_8$ also together represent a ring-forming propylene group or hydroxypropylene group, or

Z represents a straight-chain or branched lower hydroxyalkylamino radical, whenever $R_1$ represents a monomethoxy- or a monoethoxy-substituted alkyl radical having from 1 to 4 carbon atoms and/or $R_3$ represents a lower alkyl radical having from 1 to 4 carbon atoms, the lower alkyl esters thereof and the salts thereof with physiologically tolerable bases, characterised in that an amine of the general formula II

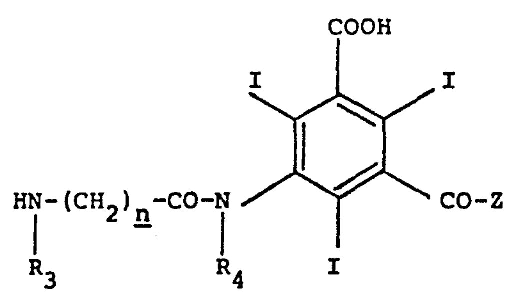

(II),

in which $R_3$, $R_4$ X and *n* have the meanings given in claim 1, is reacted with a reactive functional carboxylic acid derivative of the general formula III

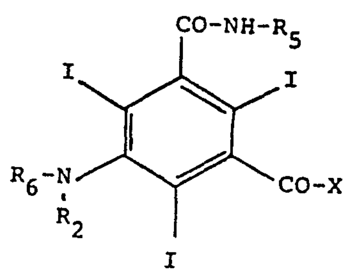

(III),

in which

$R_2$ and $R_5$ have the meanings given in claim 1, —CO—X represents a reactive carboxy group derivative or a reactive carboxylic acid ester group derivative in which X is a reactive radical, and $R_6$ represents a hydrogen atom or a —CO—$R_1$ radical and, in the case of $R_6$ = hydrogen, the aromatic amiono group is acylated with a reactive acid derivative $R_1$—CO—X in which X is a reactive radical and $R_1$ has the meaning given in claim 1 and/or an amino gorup is alkylated and/or the physiologically tolerable salts with bases or the lower alkyl esters are formed.